# EUROPEAN PATENT APPLICATION

(11) **EP 3 453 339 A1**
(43) Date of publication of application: **13.03.2019**
(21) Application number: 18193792.1
(22) Date of filing: 11.09.2018
(51) Int. Cl.: A61B 17/072

(54) **STAPLE RELOAD WITH INTEGRAL TISSUE STOPS**

(30) Priority: 12.09.2017 US 201762557707 P; 21.08.2018 US 201816107284
(71) Applicant: Covidien LP, Mansfield, MA 02048 (US)
(72) Inventor: BUDHABHATTI, Sachin P., Unionville, Connecticut 06085 (US); BRONSON, Dwight, Cheshire, Connecticut 06410 (US)
(74) Representative: Maschio, Antonio

(57) **Abstract**

A staple reload includes a body having spaced side walls, an upper surface that extends between the upper ends of the spaced side walls, and tissue stops that extend upwardly from the side walls to a position above the upper surface of the body. A portion of the upper surface of the body positioned distally of the tissue stops defines a tissue contact surface of the body. The tissue contact surface defines rows of staple receiving slots. The tissue stops are positioned adjacent a proximal-most staple receiving slot(s) and have a distal surface that defines the proximal end of the tissue contact surface.

## Description

### CROSS-REFERENCE TO RELATED APPLICATIONS

This application claims the benefit of and priority to U.S. Provisional Patent Application Serial No. 62/557,707 filed September 12, 2017, the entire disclosure of which is incorporated by reference herein.

### BACKGROUND

### 1. Technical Description

The present disclosure is directed to a staple reload for a surgical stapling device and, more particularly to a staple reload including integrally formed tissue stops. The present disclosure is also directed to a surgical stapling device including the presently disclosed staple reload and to a kit including a surgical stapling device with a plurality of different size staple reloads.

### 2. Background of Related Art

Surgical stapling devices typically include a pair of jaws that are movable in relation to each other between spaced and clamped positions to clamp tissue to be stapled and/or transected. In some stapling devices, one of the jaws defines a channel that is configured to receive a staple reload and the other jaw supports an anvil. The staple reload is supported within the channel and includes a tissue contact surface that defines a plurality of staple receiving pockets. In some stapling devices, one of the jaws defines a tissue stop that is positioned to prevent tissue from being positioned on the tissue contact surface between the jaws at a location proximally of the staple receiving pockets. The tissue stop is supported on the jaws at a specific location to accommodate a staple reload of a particular size. Thus, the stapling device can only accommodate staple reloads of a single size.

A continuing need exists in the stapling art for a surgical stapling device that can accommodate a variety of different size staple reloads.

### SUMMARY

One aspect of the present disclosure is directed to a staple reload that includes a body including spaced side walls having upper ends, an upper surface extending between the upper ends of the spaced side walls, and tissue stops extending upwardly from the side walls to a position above the upper surface of the body. The staple reload includes a plurality of staples and the body defines a plurality of staple receiving slots. A portion of the upper surface of the body positioned distally of the tissue stops defines a tissue contact surface. Each of the plurality of staple receiving slots opens onto the tissue contact surface. Each of the plurality of staples is received within one of the plurality of staple receiving slots. The tissue stops are positioned adjacent a proximal-most staple receiving slot of the plurality of staple receiving slots and define a proximal end of the tissue contact surface.

Another aspect of the present disclosure is directed to a surgical stapling device including a tool assembly having a first jaw supporting an anvil and a second jaw supporting a staple reload. The first jaw is movable in relation to the second jaw between spaced and clamped positions. The staple reload includes a body having spaced side walls with upper ends. An upper surface of the body extends between the upper ends of the spaced side walls. Tissue stops extend from the side walls towards the first jaw to a position above the upper surface. The staple reload includes a plurality of staples and the body defines a plurality of staple receiving slots. A portion of the upper surface positioned distally of the tissue stops defines a tissue contact surface. Each of the plurality of staple receiving slots opens onto the tissue contact surface. Each of the plurality of staples is received within one of the plurality of staple receiving slots. The tissue stops are positioned adjacent a proximal-most staple receiving slot of the plurality of staple receiving slots and define a proximal end of the tissue contact surface.

In embodiments, the body defines a knife slot.

In some embodiments, the plurality of staple receiving slots is aligned in rows on the tissue contact surface and at least two of the rows are positioned on each side of the knife slot.

In certain embodiments, the tissue contact surface has a length that is less than half of an overall length of the upper surface of the body.

In embodiments, the tissue contact surface has a length that is less than two-thirds of the overall length of the upper surface of the body.

In some embodiments, each of the tissue stops includes a post defining a distal face.

In certain embodiments, the proximal-most staple receiving slot extends proximally of the distal face of the posts.

Another aspect of the present disclosure is directed to a kit including a tool assembly having a first jaw supporting an anvil and a second jaw defining a channel, and first and second staple reloads that are configured to be releasably coupled to the second jaw. Each of the first and second staple reloads has a body including spaced side walls having upper ends. An upper surface of the body extends between the upper ends of the spaced side walls. Tissue stops extend from the upper ends of the side walls towards the first jaw to a position above the upper surface of the body. The staple reload includes a plurality of staples and the body defines a plurality of staple receiving slots. Each of the plurality of staples is received within one of the plurality of staple receiving slots. A portion of the upper surface positioned distally of the tissue stops defines a tissue contact surface. Each of the plurality of staple receiving slots opens onto the tissue contact surface. The upper surface of the body of the first reload has a first length and the upper surface of the body of the second reload has a second length wherein the first length is equal to the second length. The tissue contact surface of the body of the first reload has a third length and the tissue contact surface of the body of the second reload has a fourth length, wherein the third length is greater than the fourth length.

In embodiments, the fourth length is less than half of first and second lengths of the upper surface of the body of the first and second staple reloads.

In some embodiments, the fourth length is less than two thirds of first and second lengths of the upper surface of the body of the first and second staple reloads.

### BRIEF DESCRIPTION OF THE DRAWINGS

Various embodiments of the presently disclosed surgical stapling device are described herein below with reference to the drawings, wherein:
FIG. 1 is an exploded, side perspective view of an exemplary embodiment of the presently disclosed surgical stapling device including three different size staple reloads;
FIG. 2 is an enlarged view of the indicated area of detail shown in FIG. 1;
FIG. 3 is a side perspective view of the surgical stapling device shown in FIG. 1 in a clamped position with an 80mm staple reload secured to a cartridge channel of a cartridge receiving half-section of the surgical stapling device;
FIG. 4 is a side perspective view of the surgical stapling device shown in FIG. 1 in the clamped position with a 60mm staple reload secured to the cartridge channel of the cartridge receiving half-section of the surgical stapling device;
FIG. 5 is a side perspective view of the surgical stapling device shown in FIG. 3 clamped about tissue with the 80mm staple reload secured to the cartridge channel of the cartridge receiving half-section of the surgical stapling device;
FIG. 6 is a side perspective view of another embodiment of the presently disclosed surgical stapling device with the tool assembly of the surgical stapling device in an open position; and
FIG. 7 is an enlarged view of the indicated area of detail shown in FIG. 6.

### DETAILED DESCRIPTION OF EMBODIMENTS

The presently disclosed surgical stapling device will now be described in detail with reference to the drawings in which like reference numerals designate identical or corresponding elements in each of the several views. However, it is to be understood that the disclosed embodiments are merely exemplary of the disclosure and that the presently disclosed surgical stapling device may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

In this description, the term "proximal" is used generally to refer to that portion of the device that is closer to a clinician, while the term "distal" is used generally to refer to that portion of the device that is farther from the clinician. In addition, the term "endoscopic" is used generally to refer to endoscopic, laparoscopic, arthroscopic, and/or any other procedure conducted through small diameter incision or cannula. Finally, the term "clinician" is used generally to refer to medical personnel including doctors, nurses, and support personnel. It is also to be understood that all spatial references, such as, for example, horizontal, vertical, top, upper, lower, bottom, left and right, are for illustrative purposes only and can be varied within the scope of the disclosure. For example, the references "upper" and "lower" are relative and used only in the context to the other, and are not necessarily "superior" and "inferior".

The presently disclosed surgical stapling device includes a first jaw, a second jaw that is movable in relation to the first jaw between an open position and a clamped position, and a staple reload. The staple reload includes a body that defines spaced side walls having upper ends, an upper surface that extends between the upper ends of the spaced side walls, and tissue stops that extend from the side walls towards the first jaw to a position above the upper surface of the body. A portion of the upper surface of the body positioned distally of the tissue stops defines a tissue contact surface of the body. The body also defines a plurality of staple receiving slots. Each of the staple receiving slots opens onto the tissue contact surface and supports a staple. The tissue stops are positioned adjacent the proximal-most staple receiving slot(s) and have a distal surface that defines the proximal end of the tissue contact surface. The first jaw of the surgical stapling device includes a channel that is configured to receive the staple reload such that the staple reload can be selectively removed from the channel and replaced with a different staple reload. The staple receiving slots are aligned in rows, e.g., 2 or more, that can extend along any desired length of the tissue contact surface of the body of the staple reload. As such, the staple reload can includes rows of staple receiving slots of any desirable length, e.g., 60mm, 80mm, 100mm, etc. By incorporating the tissue stops directly onto the body of the staple reload, a stapling device with a first jaw having a channel of a fixed length can be used to support staple reloads having a variety of different length tissue contact surfaces and staple rows.

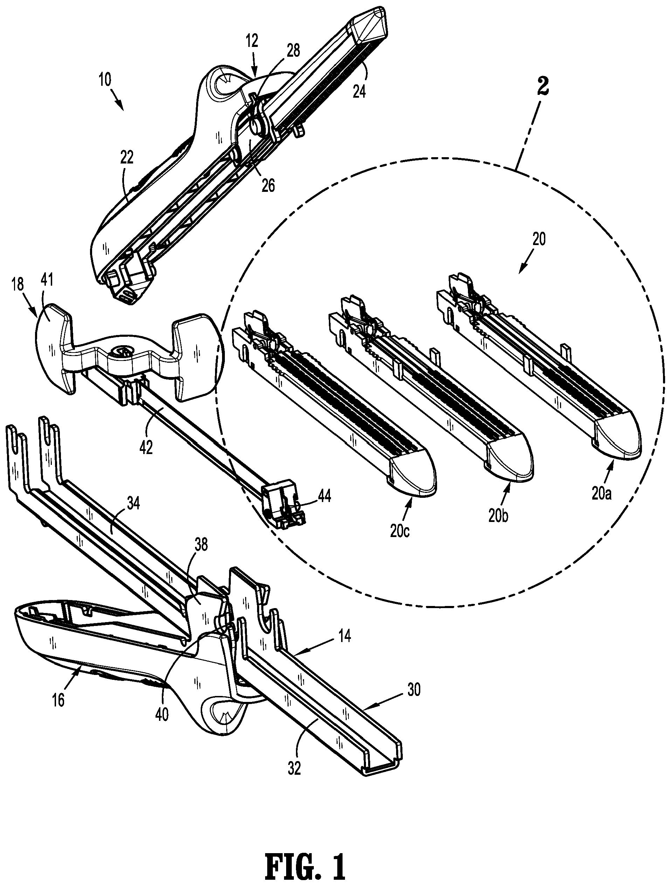

Referring to FIG. 1, in an exemplary embodiment of the presently disclosed surgical stapling device, shown generally as 10, the stapling device 10 includes a first jaw or anvil half-section 12, a second jaw or cartridge receiving half-section 14, a clamping lever 16, and a firing unit 18. Staple reloads 20 are provided to be received on the cartridge receiving half-section. In embodiments, any one or all of the components of the stapling device 10 can be disposable or formed of a biocompatible material suitable for resterilization and repeated use, e.g., stainless steel. Typically, the firing unit 18 and the staple reload 20 are formed as disposable assemblies.

The anvil half-section 12 includes a proximal handle portion 22, a distal anvil portion 24, and a central body portion 26. The central body portion 26 includes lateral support members 28 (only one is shown) that extend radially outwardly from opposite sides of the anvil half-section 12.

The cartridge receiving half-section 14 includes a channel 30 having a fixed length that has a distal portion 32 and a proximal portion 34. The distal portion 32 is dimensioned to receive a staple reload 20. The proximal portion 34 of the channel 30 is dimensioned to receive the firing unit 18.

The clamping lever 16 is pivotally supported on the cartridge receiving half-section 14 and includes a distal flange member 38. The distal flange member 38 defines recesses 40 that are configured to capture the lateral support members 28 of the central body portion 26 of the anvil half-section 12 such that pivotal movement of the clamping lever 16 towards the cartridge receiving half-section 14 moves the cartridge receiving half-section 14 in relation to the anvil half-section 12 between spaced and clamped positions.

The firing unit 18 of the stapling device 10 includes an actuator 41. The actuator 41 is attached to cam bars 42 that are positioned to be advanced through a staple reload 20 when the actuator 41 is advanced along the proximal portion 34 of the channel 30 of the cartridge receiving half-section 14 to eject staples 45 from the staple reload 20. A knife bar 44 may also be provided in the firing unit 18 to transect tissue. U.S. Publication No. 2013/0037595 discloses detailed embodiments of components suitable for use with the presently disclosed surgical stapling device 10 and is incorporated herein in its entirety by reference.

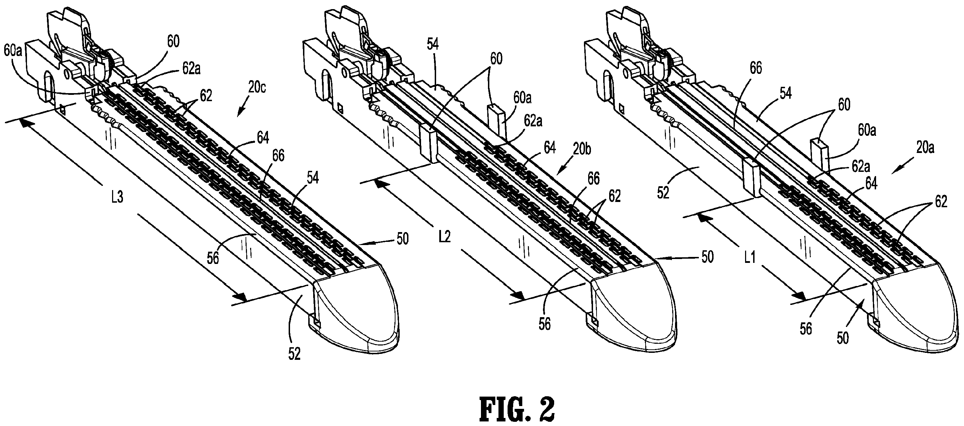

Referring also to FIG. 2, the staple reload 20 can be of a variety of different sizes. For example, a staple reload 20a has a tissue contact surface with staple rows having a length of about 60mm, a staple reload 20b has a tissue contact surface with staple rows having a length of about 80mm, and a staple reload 20c has a tissue contact surface with staple rows having a length of about 100mm. It is envisioned that the staple reloads 20a-c can have tissue contact surfaces and staple rows that have any desirable length. Each of the staple reloads 20a-c is substantially identical in most respects. The differences are described below.

Each of the staple reloads 20a-c includes a body 50 having spaced side walls 52, an upper surface 54 (as viewed in FIG. 2) extending between upper ends 56 of the spaced side walls 52, and tissue stops 60 that extend upwardly from the side walls 52 to a position above the upper surface 54 of the body 50. The tissue stops 60 can be fixedly secured to the body 50 or monolithically formed with the body 50. The body 50 defines a plurality of staple receiving slots 62. A distal portion of the upper surface 54 positioned distally of the tissue stops 60 defines a tissue contact surface 64. The portion of the upper surface 54 of the body 50 proximal of the tissue stops 60 does not engage tissue. Each of the plurality of staple receiving slots 62 opens onto the tissue contact surface 64 of the body 50 and receives a staple (not shown).

In embodiments, the body 50 of the staple reload 20a-c also defines a central knife slot 66. In some embodiments, the staple receiving slots 62 are configured in rows that are positioned on opposite sides of the central knife slot 66. The body 50 may include one or more rows on each side of the central knife slot 66, e.g., 2 or 3 rows.

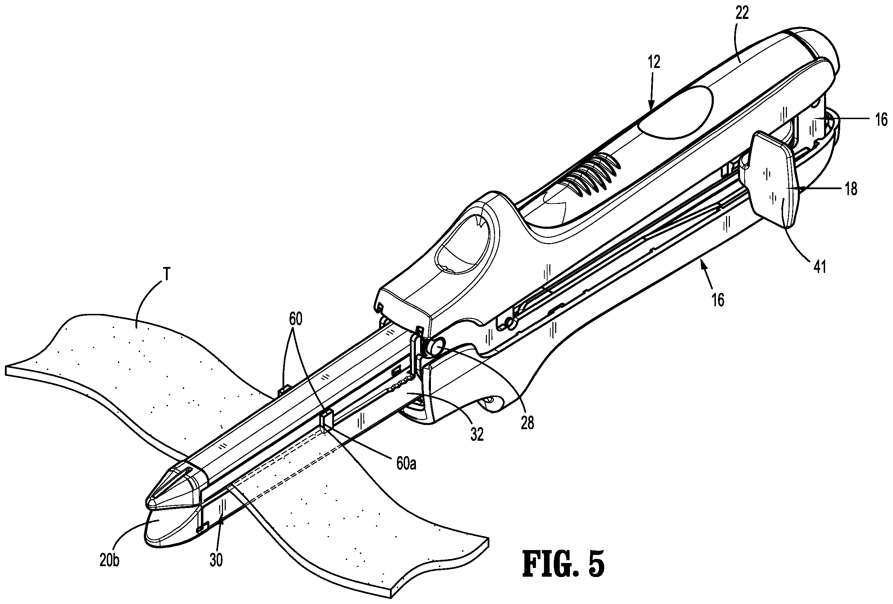

The tissue stops 60 have a distal surface 60a that is positioned to abut tissue "T" (FIG. 5) to prevent the tissue "T" from moving proximally beyond the tissue contact surface 64 and the rows of staples. In embodiments, the proximal-most staple receiving slots 62a are aligned with the distal surface 60a of the tissue stops 60 or positioned slightly proximal of the distal face 60a of the tissue stop 60. These configurations ensure that tissue "T" (FIG. 5) that is transected by the stapling device 10 is sutured by staples (not shown).

In embodiments, the tissue stops 60 are configured as rectangular posts that have a rectangular distal surface 60a that abuts the tissue "T" (FIG. 5) to prevent the tissue "T" from moving proximally beyond the distal surface 60a of the tissue stops 60. Alternately, the tissue stops 60 can assume any of a variety of different configurations suitable to prevent movement of the tissue "T" proximally of the tissue stops 60.

As illustrated in FIG. 2, the tissue stops 60 can be selectively positioned anywhere along the length of the body 50 to define any desirable length tissue contact surface 54 on the staple reload 20. For example, on the staple reload 20a, the tissue stops 60 are positioned on the body 50 to define a tissue contact surface 64 having a length "L1" of about 60mm. In contrast, on the staple reload 20b, the tissue stops 60 are positioned on the body 50 to define a tissue contact surface 64 having a length "L2" of about 80mm, and on the staple reload 20c, the tissue stops 60 are positioned on the body 50 to define a tissue contact surface 64 having a length "L3" of about 100mm. The tissue stops 60 can be positioned on the body 50 of the staple reloads 20a-c at any desirable position to define a tissue contact surface 64 of any desirable length. As discussed above, the length of the tissue contact surface 64 corresponds substantially to the length of the rows of staple receiving slots 62.

In embodiments, the tissue contact surface 64 has a length that is less than half of the overall length of the upper surface 54 of the body 50 of the staple reload 20. See the staple reload 20a. In other embodiments, the tissue contact surface 64 of the body 50 has a length that is less than two thirds of the overall length of the upper surface 54 of the body 50 of the staple reloads 20. See the staple reload 20b. In certain embodiments, the body 50 of the staple reload 20c has a length that is substantially equal to the overall length of the upper surface 54 of the body 50 of the staple reloads 20.

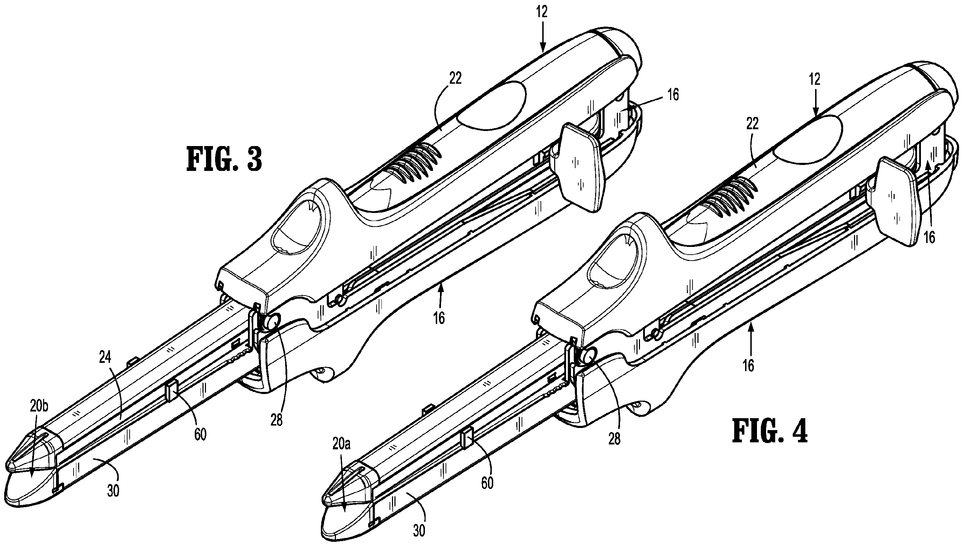

Referring to FIGS. 3-5, when the staple reload 20a-c is supported within the channel 30 of the cartridge receiving half-section 14, the tissue stops 60 extend upwardly towards the anvil half-section 12. The tissue stops 60 should be dimensioned to prevent tissue "T" (FIG. 5) from passing between the anvil half-section 12 and the cartridge receiving half-section 14 proximally beyond the tissue stops 60 when the anvil half-section 12 and cartridge receiving half-section 14 of the stapling device 10 are in the spaced position. When the anvil half-section 12 and cartridge receiving half-section 14 of the stapling device 10 are moved to the clamped position (FIG. 5), the tissue stops 60 extend upwardly along an outer surface of the distal anvil portion 24 of the anvil half-section 14.

It is envisioned that the presently disclosed surgical stapling device 10 can be provided in a kit with a plurality of staple reloads 20a-c, such as shown in FIG. 1, wherein the staple reloads 20 each have a tissue contact surface 64 (and rows of staples) that has a different length than the other staple reloads 20. For example, the kit may include a surgical stapling device 10 with two or more staple reloads 20 that have tissue contact surfaces 64 (FIG. 2) with different lengths and thus, rows of staples with different lengths.

In some embodiments, the kit includes first and second staple reloads 20a-b. The upper surface of the body 50 of the first reload 20a has a first length and the upper surface of the body of the second reload 20b has a second length, wherein the first length is equal to the second length. In this embodiment, the tissue contact surface 64 of the body 50 of the first reload 20a has a third length and the tissue contact surface 64 of the body 50 of the second reload 20b has a fourth length, wherein the fourth length "L2" is greater than the third length "L1".

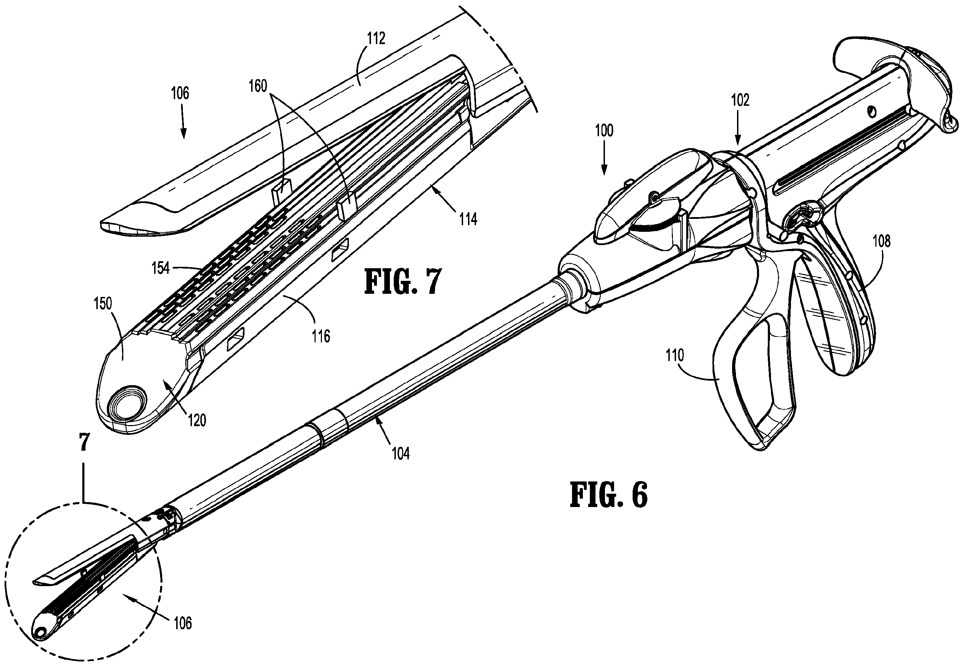

FIGS. 6 and 7 illustrate another exemplary embodiment of the presently disclosed stapling device shown generally as 100. In contrast to the stapling device 10 (FIG. 1) which is an open-type surgical stapling device, the stapling device 100 is an endoscopic surgical stapling device. The stapling device 100 includes a handle assembly 102, an elongate body 104, and a tool assembly 106. The handle assembly 102 includes a stationary handle 108 and a pivotal trigger 110. The elongate body 104 extends distally from the handle assembly 102 and has a distal end that supports the tool assembly 108.

The tool assembly 106 includes an anvil assembly 112 and a cartridge assembly 114 that are movable in relation to each other between spaced and clamped positions. For a detailed description of the components of an endoscopic surgical stapling device similar to stapling device 100, see US. Patent No. 8,418,904 which is incorporated herein by reference in its entirety.

The stapling device 100 is similar to the stapling device 10 in that the cartridge assembly 114 of the tool assembly 106 includes a channel 116 and a staple reload 120. The staple reload 120 is supported within the channel 116 such that the staple reload 120 can be selectively removed from the channel 116 and replaced. The staple reload 120 includes a body 150 that supports tissue stops 160. As discussed above, the tissue stops 160 can be fixedly secured to the body 150 or monolithically formed with the body 150.

Similar to the tissue stops 60 described above (FIG. 1), the tissue stops 160 can be selectively positioned anywhere along the length of the body 150 of the staple reload 120 to define any desirable length tissue contact surface 154 on the staple reload 120. This allows the channel 116 of the tool assembly 106 to receive staple reloads 120 that have the same overall length but have different length tissue contact surfaces 164 (and different length rows of staples). As such, the stapling device 100 can be used with different size staple reloads.

Persons skilled in the art will understand that the devices and methods specifically described herein and illustrated in the accompanying drawings are non-limiting exemplary embodiments. For example, although the bodies of the staple reloads are illustrated as being substantially linear, the bodies of the staple reloads could be non-linear or curved. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described embodiments. Accordingly, the disclosure is not to be limited by what has been particularly shown and described, except as indicated by the appended claims.

The invention may be described by reference to the following numbered paragraphs:-
1. A staple reload comprising:
   a body including spaced side walls having upper ends, an upper surface extending between the upper ends of the spaced side walls, and tissue stops extending upwardly from the side walls to a position above the upper surface, the body defining a plurality of staple receiving slots, a portion of the upper surface positioned distally of the tissue stops defining a tissue contact surface, each of the plurality of staple receiving slots opening onto the tissue contact surface; and
   a plurality of staples, each of the plurality of staples being received within one of the plurality of staple receiving slots, wherein the tissue stops are positioned adjacent a proximal-most staple receiving slot of the plurality of staple receiving slots and define a proximal end of the tissue contact surface.
2. The staple reload of paragraph 1, wherein the body defines a knife slot.
3. The staple reload of paragraph 2, wherein the plurality of staple receiving slots are aligned in rows on the tissue contact surface, at least two of the rows being positioned on each side of the knife slot.
4. The staple reload of paragraph 1, wherein the tissue contact surface has a length that is less than half of an overall length of the upper surface of the body.
5. The staple reload of paragraph 1, wherein the tissue contact surface has a length that is less than two-thirds of an overall length of the upper surface of the body.
6. The staple reload of paragraph 1, wherein each of the tissue stops includes a post defining a distal face.
7. The staple reload of paragraph 6, wherein the proximal-most staple receiving slot extends proximally of the distal face of the posts.
8. A surgical stapling device comprising:
   a tool assembly including a first jaw supporting an anvil and a second jaw supporting a staple reload, the first jaw being movable in relation to the second jaw between spaced and clamped positions, the staple reload including:
   a body including spaced side walls having upper ends, an upper surface extending between the upper ends of the spaced side walls, and tissue stops extending from the side walls towards the first jaw to a position above the upper surface, the body defining a plurality of staple receiving slots, a portion of the upper surface positioned distally of the tissue stops defining a tissue contact surface, each of the plurality of staple receiving slots opening onto the tissue contact surface; and
   a plurality of staples, each of the plurality of staples being received within one of the plurality of staple receiving slots, wherein the tissue stops are positioned adjacent a proximal-most staple receiving slot of the plurality of staple receiving slots and define a proximal end of the tissue contact surface.
9. The surgical stapling device of paragraph 8, wherein the body defines a knife slot.
10. The surgical stapling device of paragraph 8, wherein the plurality of staple receiving slots are
   aligned in rows on the tissue contact surface, at least two of the rows being positioned on each side of the knife slot.
11. The staple reload of paragraph 8, wherein the tissue contact surface has a length that is less than half of an overall length of the upper surface of the body.
12. The staple reload of paragraph 8, wherein the tissue contact surface has a length that is less than two-thirds of an overall length of the upper surface of the body.
13. The staple reload of paragraph 8, wherein each of the tissue stops includes a post defining a distal face.
14. The staple reload of paragraph 8, wherein the proximal-most staple receiving slot is positioned proximally of the distal face of the posts.
15. A kit comprising:
   a tool assembly including a first jaw supporting an anvil and a second jaw defining a channel; and
   first and second staple reloads configured to be releasably coupled to the second jaw, each of the first and second staple reloads having a body including spaced side walls having upper ends, an upper surface extending between the upper ends of the spaced side walls, tissue stops extending from the side walls towards the first jaw to a position above the upper surface, and a plurality of staples, the body defining a plurality of staple receiving slots, a portion of the upper surface positioned distally of the tissue stops defining a tissue contact surface, each of the plurality of staple receiving slots opening onto the tissue contact surface, each of the plurality of staples being received within one of the plurality of staple receiving slots;
   wherein the upper surface of the body of the first reload has a first length and the upper surface of the body of the second reload has a second length, the first length being equal to the second length, and the tissue contact surface of the body of the first reload has a third length and the tissue contact surface of the body of the second reload has a fourth length, the third length being greater than the fourth length.
16. The kit of paragraph 15, wherein the fourth length is less than half of first and second lengths of the upper surface of the body of the first and second staple reloads.
17. The kit of paragraph 15, wherein the fourth length is less than two thirds of first and second lengths of the upper surface of the body of the first and second staple reloads.

## Claims

1. A staple reload comprising:
a body including spaced side walls having upper ends, an upper surface extending between the upper ends of the spaced side walls, and tissue stops extending upwardly from the side walls to a position above the upper surface, the body defining a plurality of staple receiving slots, a portion of the upper surface positioned distally of the tissue stops defining a tissue contact surface, each of the plurality of staple receiving slots opening onto the tissue contact surface; and
a plurality of staples, each of the plurality of staples being received within one of the plurality of staple receiving slots, wherein the tissue stops are positioned adjacent a proximal-most staple receiving slot of the plurality of staple receiving slots and define a proximal end of the tissue contact surface.

2. The staple reload of claim 1, wherein the body defines a knife slot.

3. The staple reload of claim 2, wherein the plurality of staple receiving slots are aligned in rows on the tissue contact surface, at least two of the rows being positioned on each side of the knife slot.

4. The staple reload of any preceding claim, wherein the tissue contact surface has a length that is less than half of an overall length of the upper surface of the body.

5. The staple reload of any preceding claim, wherein the tissue contact surface has a length that is less than two-thirds of an overall length of the upper surface of the body.

6. The staple reload of any preceding claim, wherein each of the tissue stops includes a post defining a distal face; preferably wherein the proximal-most staple receiving slot extends proximally of the distal face of the posts.

7. A surgical stapling device comprising:
a tool assembly including a first jaw supporting an anvil and a second jaw supporting a staple reload, the first jaw being movable in relation to the second jaw between spaced and clamped positions, the staple reload including:
a body including spaced side walls having upper ends, an upper surface extending between the upper ends of the spaced side walls, and tissue stops extending from the side walls towards the first jaw to a position above the upper surface, the body defining a plurality of staple receiving slots, a portion of the upper surface positioned distally of the tissue stops defining a tissue contact surface, each of the plurality of staple receiving slots opening onto the tissue contact surface; and
a plurality of staples, each of the plurality of staples being received within one of the plurality of staple receiving slots, wherein the tissue stops are positioned adjacent a proximal-most staple receiving slot of the plurality of staple receiving slots and define a proximal end of the tissue contact surface.

8. The surgical stapling device of claim 7, wherein the body defines a knife slot.

9. The surgical stapling device of claim 7 or 8, wherein the plurality of staple receiving slots are aligned in rows on the tissue contact surface, at least two of the rows being positioned on each side of the knife slot.

10. The surgical stapling device of any of claims 7 to 9, wherein the tissue contact surface has a length that is less than half of an overall length of the upper surface of the body.

11. The surgical stapling device of any of claims 7 to 9, wherein the tissue contact surface has a length that is less than two-thirds of an overall length of the upper surface of the body.

12. The surgical stapling device of any of claims 7 to 11, wherein each of the tissue stops includes a post defining a distal face; and/or wherein the proximal-most staple receiving slot is positioned proximally of the distal face of the posts.

13. A kit comprising:
a tool assembly including a first jaw supporting an anvil and a second jaw defining a channel; and
first and second staple reloads configured to be releasably coupled to the second jaw, each of the first and second staple reloads having a body including spaced side walls having upper ends, an upper surface extending between the upper ends of the spaced side walls, tissue stops extending from the side walls towards the first jaw to a position above the upper surface, and a plurality of staples, the body defining a plurality of staple receiving slots, a portion of the upper surface positioned distally of the tissue stops defining a tissue contact surface, each of the plurality of staple receiving slots opening onto the tissue contact surface, each of the plurality of staples being received within one of the plurality of staple receiving slots;
wherein the upper surface of the body of the first reload has a first length and the upper surface of the body of the second reload has a second length, the first length being equal to the second length, and the tissue contact surface of the body of the first reload has a third length and the tissue contact surface of the body of the second reload has a fourth length, the third length being greater than the fourth length.

14. The kit of claim 13, wherein the fourth length is less than half of first and second lengths of the upper surface of the body of the first and second staple reloads.

15. The kit of claim 14, wherein the fourth length is less than two thirds of first and second lengths of the upper surface of the body of the first and second staple reloads.
